# EUROPEAN PATENT SPECIFICATION

(11) **EP 2 560 752 B1**
(45) Date of publication and mention of the grant of the patent: **19.06.2019**
(21) Application number: 11714988.0
(22) Date of filing: 19.04.2011
(51) Int. Cl.: B01J 13/18, A01N 25/28, A61K 8/11, A61K 9/50

(54) **COSMETIC PREPARATION COMPRISING A CAPSULE COMPRISING AN ACTIVE INGREDIENT**
KOSMETISCHE ZUBEREITUNG MIT EINER KAPSEL MIT EINEM WIRKSTOFF
PREPARATION COSMETIQUE COMPRENANT UNE CAPSULE COMPRENANT UN INGREDIENT ACTIF

(30) Priority: 20.04.2010 EP 10160468; 20.04.2010 US 325832 P
(43) Date of publication of application: 27.02.2013
(73) Proprietor: BASF SE, 67056 Ludwigshafen am Rhein (DE)
(72) Inventor: DREHER, Jing, 67117 Limburgerhof (DE); ETTL, Roland, 68804 Altlussheim (DE); KREUSCH, Holger, 66996 Hirschthal (DE)
(74) Representative: Upschulte, Manfred Alois
(86) International application number: PCT/EP2011/056191
(87) International publication number: WO 2011/131644

(56) References cited:
- WO-A1-2005/009604
- WO-A2-96/11054
- WO-A2-2008/002637
- WO-A2-2010/013250
- US-A- 6 103 379

## Description

The present invention relates to a cosmetic preparation comprising a capsule with a core/shell structure, comprising a core which comprises at least one sparingly water-soluble or water-insoluble organic active ingredient.

The encapsulation of active ingredients is undertaken for various reasons. For example, through encapsulation it is possible to increase the storage stability of those active ingredients which are sensitive to light, oxygen or moisture. In the case of pharmaceutical active ingredients, the active ingredient release can be influenced in a targeted manner by the encapsulation. Or liquid substances can be handled following encapsulation in the form of a pourable powder. In the case of encapsulation of organic UV filters for the area of sun protection of the human skin it is ensured through the encapsulation that the contact between human skin and the organic UV filter is reduced or even prevented.

The encapsulation of organic active ingredients with metal oxide layers or the adsorption of organic active ingredients in porous metal oxides is known.

WO 2005/009604 A1 describes microcapsules with a high active ingredient content in which a core which comprises an active ingredient is surrounded by a shell, where the shell comprises an inorganic polymer.

WO 2007/093252 A1 describes UV filter capsules which comprise at least one amino-substituted hydroxybenzophenone.

WO 2009/012871 A2 describes UV filter capsules which comprise a polymeric coating, at least one sparingly soluble organic UV filter and an emollient as solvent for the sparingly soluble organic UV filter.

WO 2010/013250 A2 discloses a composition comprising microcapsules comprising a core comprising a pharmaceutically or dermatologically active agent, and a shell, comprising metal oxide nanoparticles, where said nanoparticles are joined together by a hydrolyzed and polymerized sol-gel precursor.

Despite the prior art described at the start, there is still a need for capsules which exhibit improved stability against unintended rupture of the shell or which have a denser, less porous shell in order to prevent the active ingredient from escaping. Furthermore, the capsules which can be used in the field of cosmetics should as far as possible release no skin-irritating constituents such as, for example, surfactants. Finally, the method for producing the capsules should be as widely usable as possible and easy to carry out. The method for producing the capsules should be stable both towards thermal stresses and also towards mechanical stresses.

It was therefore the object of the present invention to provide an active-ingredient-containing capsule with improved stability, or to provide an active-ingredient-containing capsule with reduced skin irritation potential, and to be able to produce the novel active-ingredient-containing capsules by a simple and robust method.

This object is achieved by a capsule with a core/shell structure, comprising a core which comprises at least one sparingly water-soluble or water-insoluble organic active ingredient, and a shell which directly surrounds the core, where the shell comprises nanoparticles of a metal oxide or semimetal oxide and these nanoparticles are joined together by at least one further metal oxide or semimetal oxide, where the further metal oxide or semimetal oxide joining the nanoparticles has been formed by hydrolysis and subsequent polycondensation of a water-insoluble or sparingly water-soluble sol-gel precursor.

The capsule according to the invention comprises preferably less than 0.1% by weight, particularly preferably less than 0.001% by weight, very particularly preferably less than 0.00001% by weight, of low molecular weight, organic surfactants, in particular no low molecular weight, organic surfactants, based on the total weight of the capsule. No surfactants means that the capsule comprises no detectable amounts of low molecular weight organic surfactants and that no low molecular weight organic surfactant has been used in the production of the capsule. The capsule according to the invention preferably also comprises no high molecular weight protective colloids, such as, for example, gelatin, modified starch or pectins.

The mass fraction of the core relative to the total mass of the capsule is usually greater than 50% by weight, preferably from 50 to 99% by weight, particularly preferably from 60 to 90% by weight. The percentages refer to a statistical mean value determined over a large number of capsules.

The capsule according to the invention having a core/shell structure comprises in the inside in each case a core which comprises at least one sparingly water-soluble or water-insoluble organic active ingredient. The core may be either liquid or solid at 20°C. If the core is a solid at 20°C, this solid may be crystalline, partially crystalline or amorphous. If the core is a liquid at 20°C, this liquid may be homogeneous phase or a suspension. Preferably, the core of the capsule according to the invention is a liquid at 20°C.

The core in the inside of a capsule according to the invention consists preferably to more than 50% by weight, particularly preferably to more than 60% by weight, very particularly preferably to more than 80% by weight, in particular to more than 90% by weight, of at least one sparingly water-soluble or water-insoluble organic active ingredient, based on the mass of the core. In the case of an active ingredient which is present in liquid form during the production method prior to the encapsulation step, since it is melted for example as a result of the introduction of heat or is already liquid at 20°C, the core consists preferably exclusively of the sparingly water-soluble or water-insoluble active ingredient.

On account of its composition, the core preferably exhibits hydrophobic properties, i.e. the core is only sparingly water-soluble or water-insoluble.

The capsules according to the invention ordinarily have an average particle size (d50 value) of less than 1000 µm, preferably an average particle size of from 0.05 µm to 100 µm, particularly preferably a particle size of from 0.5 µm to 20 µm, in particular from 1 µm to 10 µm.

The d50 value is defined as being that 50% by weight of the particles have a diameter which is less than the value which corresponds to the d50 value, and 50% by weight of the particles have a diameter which is larger than the value which corresponds to the d50 value. The d50 value can be read off from a particle size distribution curve, as can be generated, for example, by means of light scattering according to ISO 13320-1 (e.g. Microtrac S3500 Bluewave from Microtrac).

Preferably, a capsule according to the invention has a particle size of from 0.5 to 20 µm, in particular from 1 µm to 10 µm.

The shells of the capsules according to the invention ordinarily have an average shell thickness of from 1 to 2000 nm, preferably from 1 to 200 nm. The ratio between the average thickness of the shell and the average diameter of the capsule is preferably from 1:50 to 1:500, particularly preferably from 1:100 to 1:200.

The average particle size of the capsules and the thickness of the shells can be determined by means of TEM (transmission electron microscopy). The average particle size can be determined using the methods of light scattering (static and dynamic light scattering).

The shape of the cores in the capsules according to the invention is arbitrary and can be, for example, irregular or spherical, preferably spherical.

In the core of the capsule according to the invention, an organic UV filter selected from.

Octocrylene, Ethylhexyl Methoxycinnamate, Ethylhexyl Triazone, Diethylamino Hydroxybenzoyl Hexyl Benzoate, Methylene Bis-Benzotriazolyl Tetramethylbutylphenol or Bis-Ethylhexyloxyphenol Methoxyphenyl Triazine, and mixtures of these UV filters is used as sparingly water-soluble or water-soluble organic active ingredient.

In principle, the sparingly water-soluble or water-insoluble organic active ingredient may be a liquid or a solid at 20°C, where the solid itself may also be present in a suitable lipophilic solvent, such as an oil, in dissolved form or as suspension. Preferably, the sparingly water-soluble or water-insoluble organic active ingredient used in the capsule according to the invention is a liquid at 20°C.

Besides the sparingly water-soluble and water-insoluble active ingredient, the core of the capsule according to the invention can also comprise hydrophobic auxiliaries such as oils or solvents which are usually used in the respective fields of application. In the case of cosmetic active ingredients, like the preferred UV filters, the sparingly water-soluble or water-insoluble organic active ingredient can be dissolved or suspended in typical oil components, as are used in cosmetics.

Customary oil components in cosmetics are, for example, paraffin oil, glyceryl stearate, isopropyl myristate, diisopropyl adipate, cetylstearyl 2-ethylhexanoate, hydrogenated polyisobutene, vaseline, caprylic/capric triglycerides, microcrystalline wax, lanolin and stearic acid. However, this list is exemplary and not exhaustive.

Particular preference is given to those sparingly water-soluble or water-insoluble organic active ingredients which are soluble or suspendable in the water-insoluble or sparingly water-soluble sol-gel precursor which is used for constructing the shell of the capsule according to the invention.

The shell of the capsule according to the invention which directly surrounds the core comprises nanoparticles of a metal oxide or semimetal oxide, where these nanoparticles are joined together by at least one further metal oxide or semimetal oxide, where the further metal oxide or semimetal oxide joining the nanoparticles has been formed by hydrolysis and subsequent polycondensation of a water-insoluble or sparingly water-soluble sol-gel precursor.

According to the invention, the nanoparticles of a metal oxide or semimetal oxide usually have an average particle size of from 3 nm to 500 nm, preferably from 5 nm to 300 nm, particularly preferably from 5 nm to 150 nm, very particularly preferably 10 nm to 100 nm. The particle size of the nanoparticles can be determined by known methods, for example by means of TEM (transmission electron microscopy) or using the methods of light scattering (static and dynamic light scattering).

The nanoparticles of a metal oxide or semimetal oxide used according to the invention are preferably approximately spherical.

Suitable metal oxides or semimetal oxides for the nanoparticles are in particular those oxides which are sparingly soluble in water. Examples of preferred metal oxides or semimetal oxides suitable according to the invention are TiO₂, ZrO₂, HfO₂, Fe₂O₃, ZnO, Al₂O₃ and SiO₂. Particular preference is given to silicon dioxide (SiO₂), in particular in the form of a silica gel.

The nanoparticles of a metal oxide or semimetal oxide used according to the invention preferably have a charged, particularly preferably a negatively charged, surface and are thereby stabilized against aggregation. Particular preference is given to those nanoparticles which are stabilized against aggregation at a pH greater than 8, in particular in a pH range from 9 to 10.

The nanoparticles of a metal oxide or semimetal oxide used according to the invention are particularly preferably nanoparticles of silica gel, in particular colloidal silica gel, where the particles are approximately spherical, nonporous and dispersible in water. In particular, these particles have a dense core and a surface covered with silanol groups (Si-OH). To prevent aggregation, either some of the silanol groups on the silica gel surface are deprotonated through reaction with a base, i.e. are anionically modified, or are cationically modified through reaction with Al³⁺ ions. According to the invention, preference is given to using anionically modified silica gel nanoparticles.

Nanoparticles of silica (silica gel) are available for example from Grace under the name LUDOX in the form of aqueous dispersions. The surfaces of these nanoparticles of the silica gel have, as described above, a negative charge or a positive charge in order to prevent aggregation of the nanoparticles with one another. According to the invention, those nanoparticles of silica gel, the surface of which is negatively charged (anionic types) have proven to be particularly suitable. In the case of the anionic silica gel types, sodium cations or ammonium cations usually serve as counterions to the negatively charged surface.

The further metal oxide or semimetal oxide present in the capsule according to the invention, which has been formed by hydrolysis and subsequent polycondensation of a water-insoluble or sparingly water-soluble sol-gel precursor and joins the nanoparticles with one another, is usually an oxide that is sparingly soluble in water. Examples of preferred metal oxides or semimetal oxides suitable according to the invention are TiO₂, ZrO₂, HfO₂, ZnO, Al₂O₃ and SiO₂. Particular preference is given to silicon dioxide (SiO₂), in particular in the form of a silica gel.

Particular preference is given to a capsule according to the invention, where the metal oxide or semimetal oxide of the nanoparticles and the metal oxide or semimetal oxide formed by hydrolysis of the water-insoluble or sparingly water-soluble sol-gel precursor are in each case silicon dioxide, in particular a silica gel.

Water-insoluble or sparingly water-soluble sol-gel precursors which can be used according to the invention are described, for example, in WO 2005/009604 A1 page 10, line 1 to page 11, line 11.

Water-insoluble or sparingly water-soluble sol-gel precursors which can be used are preferably metal or semimetal alkoxide monomers, metal esters, semimetal esters or partially hydrolyzed and partially condensed polymers or mixtures thereof. These sol-gel precursors are preferably homogeneously miscible with the organic active ingredient. Particularly preferably, the organic active ingredient can be homogeneously dissolved in the sol-gel precursor, or the sol-gel precursor and the organic active ingredient form a homogeneous solution, it being necessary, if appropriate, to warm or heat the mixture. Alternatively, it is also possible to use a suitable organic solvent which is likewise immiscible or only poorly miscible with water, in order to provide a homogeneous solution comprising the active ingredient and the sol-gel precursor.

Suitable and preferred sol-gel precursors are compounds of the formula M(R)ₙ(P)ₘ, in which M is a metal or semimetal, such as, for example, Si, Ti, Zr, Hf, Zn or Al, preferably Si, R is a hydrolyzable substituent and n is an integer from 2 to 4, P is a nonpolymerizable substituent and m is an integer from 0 to 4 or a partially hydrolyzed or partially condensed polymer thereof or some mixture thereof. During the hydrolysis of the M-R bond, RH is cleaved off and forms a M-OH bond. In a subsequent condensation reaction, two M-OH fragments react to form a M-O-M group with the elimination of water. Examples of hydrolyzable substituents R are alkoxy radicals, such as, for example, methanolate, ethanolate or isopropanolate, or carboxylate radicals, such as, for example, acetate, palmitate or stearate.

In particular, preference is given to using tetraethyl orthosilicate (tetraethoxysilane or Si(OEt)₄) or a partially hydrolyzed or partially condensed polymer thereof or a mixture thereof in the method described above. Very particular preference is given to using tetraethyl orthosilicate as sol-gel precursor.

The shell of the capsules according to the invention is preferably transparent, especially in the case of a UV filter as active ingredient.

The present disclosure further provides a method for producing capsules with a core/shell structure, comprising in each case a core which comprises at least one sparingly water-soluble or water-insoluble organic active ingredient, and a shell which directly surrounds the core, where the shell comprises nanoparticles of a metal oxide or semimetal oxide and these nanoparticles are joined together by at least one further metal oxide or semimetal oxide, where the further metal oxide or semimetal oxide joining the nanoparticles has been formed by hydrolysis and subsequent polycondensation of a water-insoluble or sparingly water-soluble sol-gel precursor, comprising the steps
i) preparation of an oil-in-water emulsion by emulsifying an oil phase which comprises at least one water-insoluble or sparingly water-soluble sol-gel precursor and at least one sparingly water-soluble or water-insoluble organic active ingredient in a water phase which comprises nanoparticles of a metal oxide or semimetal oxide, using shear forces,
ii) establishment of a pH in the aqueous phase of the emulsion at a value at which the hydrolysis and the subsequent polycondensation of the water-insoluble or sparingly water-soluble sol-gel precursor to form the shell surrounding the core takes place,
   and
iii) if appropriate, purification and/or isolation of the capsules with core/shell structure produced in step ii).

Preferred embodiments as regards the sparingly water-soluble or water-insoluble active ingredient, as regards the nanoparticles of a metal oxide or semimetal oxide, as regards the water-insoluble or sparingly water-soluble sol-gel precursor, and also preferred embodiments with regard to dimensions and mass fractions of the various constituents of the capsules with core/shell structure can be found in the explanations already given at the start.

In step i), the preparation of an oil-in-water emulsion by emulsifying an oil phase which comprises at least one water-insoluble or sparingly water-soluble sol-gel precursor, and at least one sparingly water-soluble or water-insoluble organic active ingredient in a water phase which comprises nanoparticles of a metal oxide or semimetal oxide using shear forces is described.

The methods of preparing emulsions using shear forces are known in principle to the person skilled in the art. Thus, for example, fanta bowl and pestle, high-speed stirrers, high-pressure homogenizers, shakers, vibration mixers, ultrasound generators, emulsifying centrifuges, colloid mills or atomizers can be used for producing emulsions. In each case, the person skilled in the art selects the suitable method and the appropriate emulsifying tool depending on the result desired, for example the desired droplet size in the emulsion, and depending on the physiochemical properties of the selected feed materials, for example their viscosity or else their thermal resistance.

In step i) the fraction of the oil phase in the emulsion is preferably from 5 to 70% by weight, particularly preferably from 10 to 50% by weight, based on the total mass of the emulsion.

The fraction of the mass of the water-insoluble or sparingly water-soluble sol-gel precursor in the overall mass of the oil phase to be emulsified is preferably in the range from 5 to 70% by weight, particularly preferably 20 to 50% by weight, based on the sol-gel precursor tetraethoxysilane. When using a different sol-gel precursor, the mass fraction of this component relative to the overall mass of the oil phase can be calculated taking into consideration the different molar masses of the precursor compounds.

The preferred sol-gel precursor in step i) is tetraethoxysilane (Si(OEt)₄).

The nanoparticles of a metal oxide or semimetal oxide are present in the water phase before the emulsifying step usually in a concentration of from 0.01 to 4% by weight, preferably from 0.05 to 2% by weight, particularly preferably 0.1 to 1% by weight, based on the mass of the water phase.

In the case of the preferred silica gel nanoparticles, the mass of the colloidal silica gel used is preferably 1 to 15% by weight, particularly preferably 5 to 10% by weight, based on the mass of the oil phase.

The preparation of the emulsion in step i) is usually carried out in the temperature range from 1°C to 90°C, preferably from 15°C to 25°C, in particular from 19°C to 23°C. After an emulsion with the desired oil droplet size has been formed in emulsifying step i), in step ii), by establishing a suitable pH, for example by adding acid or base, the hydrolysis and polycondensation of the sol-gel precursor at the oil/water boundary is triggered.

Preferably, in step ii), a pH of from 7 to 13, particularly preferably from 7.5 to 13, in particular from 8 to 11, is established in the aqueous phase of the emulsion.

In principle, the suspension of capsules obtained at the end of step ii) can also be stabilized by adding additives such as, for example, nonionic, cationic or anionic polymers or surfactants or mixtures thereof. However, the capsules according to the invention are notable for the fact that, during their production, the use of surfactants is largely or preferably completely dispensed with.

In step iii), the capsules with a core/shell structure produced in step ii) are, if appropriate, purified and/or isolated. Appropriate purification and isolation methods are known to the person skilled in the art, such as, for example, centrifugation, filtration, evaporation of the solvents, resuspension and dialysis methods. For example, by removing the solvents, in particular by removing the water, from the aqueous suspension of the capsules it is possible to obtain a powder.

The capsules according to the invention with a core/shell structure are suitable, depending on the encapsulated active ingredient, as addition to cosmetics, pharmaceutical compositions, crop protection preparations, animal feeds, foods or nutritional supplements.

The present disclosure further provides the use of the capsules with a core/shell structure which have been described above or which have been produced by the method described above as addition to cosmetics, pharmaceutical compositions, crop protection preparations, animal feeds, foods or nutritional supplements.

The present invention further provides pulverulent or liquid preparations comprising the above-described capsules having a core/shell structure or the particles having a core/shell structure produced by the above-described method.

Besides the capsules having a core/shell structure, the pulverulent or liquid preparations usually comprise at least one of the customary additives and/or auxiliaries which are known to the person skilled in the art for the particular field of application, such as, for example, in the field of cosmetics or pharmaceutical compositions, in the crop protection sector, in the animal feed, food or nutritional supplement field.

Likewise provided by the present disclosure is the use of the above-described pulverulent or liquid preparations as addition to cosmetics, pharmaceutical compositions, crop protection preparations, animal feeds, foods or nutritional supplements.

The present invention provides cosmetics comprising the capsules according to the invention having a core/shell structure which have been described above or which have been produced by the above-described method. Particular preference is given to cosmetics for the area of skin protection against solar UV radiation.

The invention is illustrated by the following examples, although these do not limit the invention.

### Examples

### Example 1) Encapsulation of Diethylamino Hydroxybenzoyl Hexyl Benzoate (Uvinul® A Plus)

24 g of Diethylamino Hydroxybenzoyl Hexyl Benzoate (Uvinul® A Plus) were dissolved at 60°C in 48 g of tetraethoxysilane. This solution (oil phase) was cooled to room temperature (22°C). The oil phase was then homogenized with an aqueous solution of colloidal silica gel (LUDOX® LS 30) consisting of 7.2 g of silica gel (average particle size 12 nm; 220 m² surface area per g of silica gel; pH of the surface: 8), 3.6 g of sodium chloride and 288 g of water using a high-pressure homogenizer (M-110F Microfluidizer, Microfluidics) at 500 bar for 2 minutes. The formed emulsion was admixed with stirring (magnetic stirrer) with 25 g of sodium tetraborate buffer solution (pH 9) and stirred for 24 hours.

The particle size distribution of the formed capsules was determined by means of light scattering in accordance with ISO 13320-1 (Microtrac S3500 Bluewave from Microtrac): d50 = 0.5 µm.

### Example 2) Encapsulation of Ethylhexyl Triazone (Uvinul® T 150)

10 g of Ethylhexyl Triazone (Uvinul® T 150) were dissolved at room temperature (22°C) in 50 g of ethyl acetate. 40 g of tetraethoxysilane were added thereto. The oil phase prepared in this way was homogenized at room temperature (22°C) with an aqueous solution of colloidal silica gel (LUDOX® TM 40) consisting of 1.0 g of silica gel (average particle size 22 nm; 140 m² surface area per g of silica gel; pH of the surface: 9) and 290 g of water using an ultrasound rod (200 W, 7 mm) for 2 minutes. The formed emulsion was admixed with stirring (magnetic stirrer) with 25 g of sodium tetraborate buffer solution (pH 9) and stirred for 24 hours.

The particle size distribution of the formed capsules was determined by means of light scattering in accordance with ISO 13320-1 (Microtrac S3500 Bluewave from Microtrac): d50 = 1.0 µm.

### Example 3) Encapsulation of resorcinol bis(diphenylphosphate) (PDP) (not according to the invention)

24 g of resorcinol bis(diphenylphosphate) were dissolved at room temperature (22°C) in 48 g of tetraethoxysilane. The oil phase prepared in this way was homogenized at room temperature (22°C) with an aqueous solution of colloidal silica gel (LUDOX® SM 30) consisting of 7.2 g of silica gel (average particle size 7 nm; 350 m² surface area per g of silica gel; pH of the surface: 10) and 288 g of water using a high-pressure homogenizer (M-110F Microfluidizer, Microfluidics) at 500 bar for 5 minutes. The formed emulsion was admixed with stirring (magnetic stirrer) with 25 g of sodium tetraborate buffer solution (pH 9) and stirred for 24 hours.

The particle size distribution of the formed capsules was determined by means of light scattering in accordance with ISO 13320-1 (Microtrac S3500 Bluewave from Microtrac): d50 = 0.7 µm.

### Example 4) Encapsulation of linalyl acetate (not according to the invention)

10 g of linalyl acetate (boiling point: 220°C; CAS number: 115-95-7) were dissolved at room temperature (22°C) in 20 g of tetraethoxysilane and 10 g of white oil. The oil phase prepared in this way was homogenized at room temperature (22°C) with an aqueous solution of colloidal silica gel (LUDOX® LS 30) consisting of 2.0 g of silica gel (average particle size 12 nm; 220 m² surface area per g of silica gel; pH of the surface: 8) and 250 g of water using a high-pressure homogenizer (M-110F Microfluidizer, Micro-fluidics) at 500 bar for 5 minutes. The formed emulsion was admixed with stirring (magnetic stirrer) with 25 g of sodium tetraborate buffer solution (pH 9) and stirred for 24 hours. The prepared sample was called sample A.

The particle size distribution of the formed capsules of sample A was determined by means of light scattering in accordance with ISO 13320-1 (Microtrac S3500 Bluewave from Microtrac):
d50 = 0.8 µm.

10 g of linalyl acetate (boiling point: 220°C; CAS number: 115-95-7) were dissolved at room temperature (22°C) in 26 g of tetraethoxysilane and 10 g of white oil. The oil phase prepared in this way was homogenized at room temperature (22°C) with a solution of 1.0 g of cetyltrimethylammonium chloride (CTAC) in 250 g of water using a high-pressure homogenizer (M-110F Microfluidizer, Microfluidics) at 500 bar for 5 minutes. The formed emulsion was admixed with stirring (magnetic stirrer) with 25 g of sodium tetraborate buffer solution (pH 9) and stirred for 24 hours. The prepared sample was called sample B.

The particle size distribution of the formed capsules of sample B was determined by means of light scattering in accordance with ISO 13320-1 (Microtrac S3500 Bluewave from Microtrac):
d50 = 0.8 µm.

To remove the water, the samples A and B were in each case firstly spray-dried using a B-290 mini-spray dryer (Büchi, Switzerland). The spray-drying was carried out under the following conditions: entry temperature of ca. 120°C; exit temperature of ca. 55°C; use of a twin-material nozzle; use of nitrogen as spray gas.

The fine powders were dried further for 30 minutes using a HR73 moisture analyzer from Mettler Toledo at 105°C. The weight loss of the powder from sample A before and after the drying at 105°C was ca. 4.5% by weight; the weight loss of the powder from sample B before and after drying at 105°C was ca. 9.0% by weight.

The fine powders were dried further at 130°C for 15 minutes. The weight loss of the powder from sample A before and after drying at 130°C was ca. 7.8% by weight; the weight loss of the powder from sample B before and after drying at 130°C was ca. 13.2% by weight.

The powder prepared from sample A exhibits better thermal stability than the powder prepared from sample B.

## Claims

1. A cosmetic preparation, comprising a capsule with a core/shell structure, comprising a core which comprises at least one sparingly water-soluble or water-insoluble organic active ingredient selected from an organic UV filter selected from Octocrylene, Ethylhexyl Methoxycinnamate, Ethylhexyl Triazone, Diethylamino Hydroxybenzoyl Hexyl Benzoate, Methylene Bis-Benzotriazolyl Tetramethhylbutylphenol and Bis-Ethylhexyloxyphenol Methoxyphenyl Triazine, and mixtures of these UV filters, and a shell which directly surrounds the core, where the shell comprises nanoparticles of a metal oxide or semimetal oxide and these nanoparticles are joined together by at least one further metal oxide or semimetal oxide, where the further metal oxide or semimetal oxide joining the nanoparticles has been formed by hydrolysis and subsequent polycondensation of a water-insoluble or sparingly water-soluble sol-gel precursor , wherein the capsule comprises no low molecular weight, organic surfactants.

2. Cosmetic preparation according to claim 1, where the metal oxide or semimetal oxide of the nanoparticles and the metal oxide or semimetal oxide formed by hydrolysis of the water-insoluble or sparingly water-soluble sol-gel precursor are in each case silicon dioxide, in particular a silica gel.

3. Cosmetic preparation according to claims 1 or 2, where the capsule has a particle size of from 0.5 to 20 µm.

4. Cosmetic preparation according to any one of claims 1 to 3, where the nanoparticles consist of silica gel and have an average particle size of from 5 to 100 nm.

5. Cosmetic preparation according to any one of claims 1 to 4, where the capsule has a transparent shell.

## Patentansprüche

1. Kosmetische Zubereitung, umfassend eine Kapsel mit einem Kern/Schale-Aufbau, umfassend einen Kern, der mindestens einen schwer wasserlöslichen oder wasserunlöslichen organischen Wirkstoff umfasst, der aus einem organischen UV-Filter ausgewählt ist, der aus Octocrylene, Ethylhexyl Methoxycinnamate, Ethylhexyl Triazone, Diethylamino Hydroxybenzoyl Hexyl Benzoate, Methylene Bis-Benzotriazolyl Tetramethylbutylphenol oder Bis-Ethylhexyloxyphenol Methoxyphenyl Triazin sowie Mischungen dieser UV-Filter ausgewählt ist, und eine Schale, die den Kern direkt umhüllt, wobei die Schale Nanopartikel eines Metall- oder Halbmetalloxids umfasst und diese Nanopartikel durch mindestens ein weiteres Metall- oder Halbmetalloxid miteinander verbunden sind, wobei das die Nanopartikel verbindende Metall- oder Halbmetalloxid durch Hydrolyse und anschließende Polykondensation eines wasserunlöslichen oder schwer wasserlöslichen Sol-Gel-Vorläufers gebildet wurde, wobei die Kapsel keine niedermolekularen, organischen Tenside umfasst.

2. Kosmetische Zubereitung nach Anspruch 1, wobei es sich bei dem Metall- oder Halbmetalloxid der Nanopartikel und dem durch Hydrolyse des wasserunlöslichen oder schwer wasserlöslichen Sol-Gel-Vorläufers gebildeten Metall- oder Halbmetalloxid jeweils um Siliciumdioxid, insbesondere um ein Kieselgel, handelt.

3. Kosmetische Zubereitung nach Anspruch 1 oder 2, wobei die Kapsel eine Teilchengröße von 0,5 bis 20 µm aufweist.

4. Kosmetische Zubereitung nach einem der Ansprüche 1 bis 3, wobei die Nanopartikel aus Kieselgel bestehen und eine durchschnittliche Teilchengröße von 5 bis 100 nm aufweisen.

5. Kosmetische Zubereitung nach einem der Ansprüche 1 bis 4, wobei die Kapsel eine transparente Schale aufweist.

## Revendications

1. Préparation cosmétique, comprenant une capsule ayant une structure coeur/écorce, comprenant un coeur qui comprend au moins un ingrédient actif organique peu soluble dans l'eau ou insoluble dans l'eau, choisi parmi un filtre anti-UV organique choisi parmi l'octocrylène, le méthoxycinnamate d'éthylhexyle, l'éthylhexyl triazone, le benzoate de diéthylaminohydroxybenzoyl-hexyle, le méthylène-bis-benzotriazolyl tétraméthyl-butylphénol et la bis-éthylhexyloxyphénol méthoxyphényl-triazine, et des mélanges de ces filtres anti-UV, et une écorce qui entoure directement le coeur, où l'écorce comprend des nanoparticules d'un oxyde métallique ou d'un oxyde semi-métallique et ces nanoparticules sont reliées ensemble par au moins un autre oxyde métallique ou oxyde semi-métallique, où l'autre oxyde métallique ou oxyde semi-métallique reliant les nanoparticules a été formé par hydrolyse et polycondensation ultérieure d'un précurseur sol-gel insoluble dans l'eau ou peu soluble dans l'eau, où la capsule ne comprend aucun agent tensioactif organique de bas poids moléculaire.

2. Préparation cosmétique selon la revendication 1, dans laquelle l'oxyde métallique ou l'oxyde semi-métallique des nanoparticules et l'oxyde métallique ou l'oxyde semi-métallique formé par hydrolyse du précurseur sol-gel insoluble dans l'eau ou peu soluble dans l'eau sont dans chaque cas constitués de dioxyde de silicium, en particulier d'un gel de silice.

3. Préparation cosmétique selon la revendication 1 ou 2, dans laquelle la capsule possède une taille de particule allant de 0,5 à 20 µm.

4. Préparation cosmétique selon l'une quelconque des revendications 1 à 3, dans laquelle les nanoparticules sont constituées de gel de silice et possèdent une taille de particule moyenne allant de 5 à 100 nm.

5. Préparation cosmétique selon l'une quelconque des revendications 1 à 4, dans laquelle la capsule possède une écorce transparente.
